(19) Europäisches Patentamt
European Patent Office
Office européen des brevets

(11) EP 0 816 310 A2

(12) # EUROPEAN PATENT APPLICATION

(43) Date of publication:
07.01.1998 Bulletin 1998/02

(21) Application number: 97304049.6

(22) Date of filing: 11.06.1997

(51) Int Cl.6: **C07B 61/00**, C07C 209/24, C07C 209/52, C07C 209/28, C07C 209/84, C07C 213/04, C07C 209/06, B01L 3/00, B01J 19/00 // C07D307/52, C07D295/06, C07D295/08, C07D295/12, C07D211/60, C07D295/18, C07D207/32

(84) Designated Contracting States:
AT BE CH DE DK ES FI FR GB GR IE IT LI LU MC NL PT SE

(30) Priority: 14.06.1996 US 19790 P

(71) Applicant: ELI LILLY AND COMPANY
Indianapolis, Indiana 46285 (US)

(72) Inventors:
- Hahn, Patric James
  Indianapolis, Indiana 46237 (US)
- Kaldor, Stephen Warren
  Indianapolis, Indiana 46254 (US)
- Siegel, Miles Goodman
  Indianapolis, Indiana 46260 (US)
- Dressman, Bruce Anthony
  Indianapolis, Indiana 46202 (US)
- Fritz, James Erwin
  McCordsville, Indiana 46055 (US)

(74) Representative: Hudson, Christopher Mark et al
Lilly Industries Limited
European Patent Operations
Erl Wood Manor
Windlesham Surrey GU20 6PH (GB)

(54) **Scavenger assisted combinatorial process for preparing libraries of tertiary amine compounds**

(57) This invention relates to a novel solution phase process for the preparation of tertiary amine combinatorial libraries. These libraries have utility for drug discovery and are used to form wellplate components of novel assay kits.

EP 0 816 310 A2

## Description

This invention relates to a solution phase synthesis of combinatorial libraries of tertiary amine compounds. These libraries are useful for discovery of lead compounds for drug development.

Research and development expenses account for the largest outlay of capital in the drug industry. Synthesis of compounds is an expensive and time consuming phase of research and development. Historically, research chemists individually synthesized and analyzed hundreds of high purity compounds for biological screening to develop pharmaceutical leads. Although past methods brought new drugs to market, the limitations of individual synthesis and insistence on compound characterization considerably slowed the discovery process.

The need for more rapid and less expensive drug discovery methodology is increasingly important in today's competitive drug industry.

More recently, modern drug discovery has used the methods of combinatorial chemistry to generate large numbers (viz., about $10^2$ to $10^6$) of compounds generically referred to as "libraries." An important objective of combinatorial chemistry is to generate lead compounds for pharmaceutical research.

Theoretically, the total number of compounds which may be produced for a given library is limited only by the number of reagents available to form substituents on the variable positions on the library's molecular scaffold. The combinatorial process lends itself to automation, both in the generation of compounds and their biological screening.

Combinatorial chemistry may be performed in a manner where libraries of compounds are generated as mixtures with complete identification of individual compounds postponed until after positive screening results are obtained. However, a preferred form of combinatorial chemistry is "parallel array synthesis" where individual reaction products (most often individual compounds) are synthesized together, but are retained in separate vessels. For example, the library compounds are held in the individual wells of 96 well microtiter plates. Use of standardized microtiter plates or equivalent apparatus is advantageous because such an apparatus is readily manipulated by programmed robotic machinery.

Conventionally, combinatorial chemistry is conducted on a solid phase support, normally a polymer. The library, scaffold is cleavably tethered to the solid support by a chemical linker. Reactions are carried out to modify the scaffold while tethered to the solid support. In a final step, the product is cleaved and released from the solid support. A general procedure for conventional solid phase synthesis is illustrated by the following scheme where the shaded circle symbol is, for example, a high molecular weight polymer:

**Solid-Phase Synthesis:**

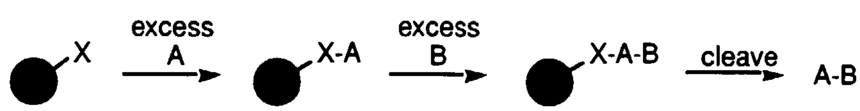


Variations in reagents (e.g., "A", "B", in the general scheme, supra.) produce the desired structural diversity. Separation of solid phase product and unreacted soluble reactant is done by simple separation techniques such as filtration, decantation, or washing. These separation solid phase synthesis techniques have general applicability to a wide variety of combinatorial reactants and lend themselves to large scale simultaneous/automated library creation.

The rate determining step in small molecule synthesis is typically not actual construction of the desired new chemical entities. Rather, the difficulty of synthesis is frequently caused by the task of isolating reaction product from unreacted starting materials, by-products or other impurities.

Unfortunately, it is not always practicable to tether a desired combinatorial scaffold to a solid support. A significant number of combinatorial reaction schemes are desirably done in solution phase. Moreover, not all desired solution phase reactions are driven to completion using near stoichiometric ratios of reactants. Frequently, one reagent is added in considerable excess to drive a solution phase reaction to completion. The result is a reaction medium with soluble product and soluble unreacted co-reactant. Consequently, traditional organic synthetic methods often require complex purification strategies which limit their use, particularly in combinatorial syntheses.

Polymeric reagents have been known and used for general chemical synthesis in various roles; such as follows:

a) The article, *Cyanoborohydride supported anion exchange resin as a selective reducing agent* by Hutchins, Robert O.: Natale, Nicholas R. and Taffer, Ira M.; J.C.S. Chem Comm., pg. 1088-9, 1978 describes various reactions including reductive amination using cyanoborohydride anion on ion-exchange resin. The spent resin reagent is removed by simple filtration and washing.

b) The article, *Synthesis and Reactivity of Polymer-Supported Reducing Agents with Chemically Modified Surfaces* by Menger, Fredric M., Hiraku, Shinozaki, and Lee, Hsueh-Chi; J. Org. Chem 1980, 45, 2724-2725 describes

borohydride and cyanoborohydride functional anion exchange resins for carbonyl group reduction. Aldehydes and ketones are reduced to form alcohols by use of polymeric reagents.

c) US Patent No. 3,873,621 describes reductive amination reactions carried out using alkali-metal and quaternary ammonium cyanoborohydrides.

d) The article, *The reduction of α,β- unsaturated nitroalkenes to nitroalkanes with borohydride supported on an ion exchange resin,* by Goudgaon, Naganna, M.; Wadgaonkar, Prakash P.; and Kabalka, George W.; Synthetic Communications, 19(5&6), 805-811 (1989) describes the use of polymer supported borohydride reagent used to reduce nitroalkenes to nitroalkanes.

e) The article, *Borohydride reducing agent derived from anion exchange resin: Selective reduction of α,β-carbonyl compound by* Sande, A.R. et. al., Tetrahedron Letters, Vol. 25, No. 32, pp 3501-3504 1984 describes the use of borohydride exchange resin for the reduction of cyclic and acyclic ketones to alcohols with the attendant advantage of simple separation by filtration to give product free of boron moiety.

f) The article, *A reductive amination/lactamization procedure using borohydride reagents* by Abdel-Magid, Ahmed F.; Harris, Brice D. and Maryanoff, Cynthia A.; Synlett, pgs. 81-3, January 1994 describes reductive amination of carbonyl compounds using sodium triacetoxyborohydride.

g) The article, *New Probes for the Study of Acylation Reactions,* by J. Rebek, D. Brown, and S. Zimmerman (Contribution No. 3481), Journal of the American Chemical Society, 97:15, p.4408, July 23, 1975, JACS 97:15 July 23, 1975 describes the use of polymer bound isocyanate to activate carboxylic acid.

h) The article, *Chemical Modification of Polymers. Borohydride Reducing Agents Derived from Anion Exchange Resins,* by H. W. Bibson and F.C. Bailey, J.C.S. Chem. Comm., p. 815, 1977 describes the preparation of an insoluble polymer bound reducing agent by reacting anion exchange resins of the quaternary ammonium type with aqueous $NaBH_4$.

i) The article, *Solid Phase Synthesis of Oligosaccharides. I. Preparation of the Solid Support. poly(p-(1-propen-3-ol-1-yl) styrene,* by J. M. Frechet and C. Schuerch, Journal of the American Chemical Society, 93:2, p. 492-496 describes the preparation of -CHO functional polymers by reacting chloromethylated resin with methyl sulfoxide and sodium bicarbonate.

j) U.S. Patent No. 3,576,870 describes the purification of dimethylacetamide by removing acetic anhydride with a basic ion exchange resin containing primary or secondary amino groups.

k) The article, *Use of Polymeric Nucleophiles for the Selective Binding and Removal of α-Methylene-γ-butyrolactone Allergens from Complex Mixtures,* by A. Cheminat and C. Benezra, Tetrahedron Letters, Vol. 21, p. 617-619 (1980) describes an amine functional polymer used as a nucleophile for removal of an α,β-unsaturated lactone electrophile.

1) The article, *Polymeric De-blocking Agents for the Fluoren-9-ylmethoxycarbonyl (FMOC) Amino-protecting Group,* by L.S. Carpino and J.R. Williams, J.C.S. Chem. Comm., p.450-451, (1978) describes the use of a resin bound piperazine to remove FMOC with subsequent scavenging of the dibenzofulvene contaminant.

m) The article, *Piperazino-Functionalized Silica Gel as a Deblocking-Scavenging Agent for the 9-Fluorenylmethyloxycarbonyl Amino-Protecting Group,* by L.A. Carpino, E.M.E. Mansour, and J. Knapczyk, J. Org. Chem., 48, p. 666-669 (1983) describes a silica bound piperazine to remove FMOC with subsequent scavenging of the dibenzofulvene contaminant.

n) The article, Preparation of High Capacity Aminomethyl-Polystyrene Resin, by C. C. Zikos and N.G. Ferderigos, Tetrahedron Letters, Vol. 36, No. 21, p. 3741-3744, 1995, describes the preparation of an amine functional resin.

o) U.S. Patent No. 5,244,582 relates to reactive groups immobilized on inorganic substrates such as glass. Such immobilized groups can be used to remove nitrosating agents in liquids, etc.

There remains a need to develop solution phase combinatorial processes for making libraries.

## Summary of the Invention

Combinatorial chemistry may be used at two distinct phases of drug development. In the discovery phase highly diverse libraries are created to find lead compounds. In a second optimization phase, strong lead compounds are much more narrowly modified to find optimal molecular configurations. The method of this invention has applicability for making a diverse library of tertiary amine compounds useful for finding new lead compounds, and directed libraries of tertiary amine compounds useful for optimizing a particular desired biological activity.

FIG. 1 is a top view of a wellplate apparatus.

FIG. 2 is a side view of a wellplate apparatus.

## Detailed Description of the Invention

### I. Definitions:

The following terms have the meaning defined below when used in this specification of the invention:

"Amine reactive substituent" means a radical symbolized by (ARS) which is selected from the group consisting of isocyanate, isothiocyanate, and acylhalide.

"Assay kit" means an assemblage of two cooperative elements, namely, (i) a wellplate apparatus, and (ii) biological assay materials.

"Biological assay materials" are materials necessary to conduct a biological evaluation of the efficacy of any library compound in a screen relevant to a selected disease state.

"Directed Library" is a collection of compounds created by a combinatorial chemistry process for the purpose of optimization of the activity of a lead compound, wherein each library compound has a common scaffold, and the library, considered in its entirety, is a collection of closely related homologues or analogues to the lead compound (compare to "Diverse library").

"Diverse library" means a library where the substituents on the combinatorial library scaffold are highly variable in constituent atoms, molecular weight, and structure and the library, considered in its entirety, is not a collection of closely related homologues or analogues (compare to "Directed library").

"Electrophile" means an electron seeking reagent having an electrophilic substituent, E.

"Lead compound" means a compound in a selected combinatorial library for which the Assay kit has revealed significant activity relevant to a selected disease state.

"Library" is a collection of compounds created by a combinatorial chemical process, said compounds having a common scaffold with one or more variable substituents.

"Library compound" means an individual reaction product (usually a single compound) in a library produced by the method of the invention.

"Parallel array synthesis" means a method of conducting combinatorial chemical synthesis of libraries wherein the individual combinatorial library reaction products are separately prepared and stored without prior or subsequent intentional mixing.

"Reaction zone" means the individual vessel location where the combinatorial chemical library compound preparation process of the invention is carried out and individual library compounds synthesized. Suitable reaction zones are the individual wells of a wellplate apparatus.

"Scaffold" means the invariant region (viz., core) of the compounds which are members of a library (viz., tertiary amine).

"Simultaneous synthesis" means making of library of compounds within one production cycle of a combinatorial method (not making all library compounds at the same instant in time).

"Solid-supported scavenger" means a reaction medium insoluble solid substance containing chemical functionality reactive with the soluble impurity (viz., usually excess reactant) desired to be removed from the reaction medium in the presence of soluble product.

"Substituents" are chemical radicals which are bonded to the scaffold through the combinatorial synthesis process. The different functional groups account for the diversity of molecules throughout the library and are selected to impart diversity of biological activity to the scaffold in the case of diverse libraries, and optimization of a particular biological activity in the case of directed libraries.

"Reagent" means a reactant, any chemical compound used in the combinatorial synthesis to place substituents on the scaffold of a library.

"Wellplate apparatus" means a structure capable of holding a plurality of library compounds in dimensionally fixed and defined positions.

"Non-interfering substituent", refers to a halo or organic (non-hydrogen) radical suitable for substitution as $R_1$, $R_2$, $R_3$ or $R_4$ in the reactants used in the process of making a combinatorial tertiary amine library. Non-interfering substituents are those that do not significantly impede the solution phase processes of the invention or interfere with the use of a solid phase scavenger in said processes. Suitable non-interfering radicals include, but are not limited to, $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, substituted phenyl, toluyl, xylenyl, biphenyl, $C_2$-$C_{12}$ alkoxyalkyl, $C_1$-$C_6$ alkylsulfinyl, $C_1$-$C_{10}$ alkylsulfonyl, -$(CH_2)_m$-O-($C_1$-$C_{10}$ alkyl), aryl, substituted aryl, substituted alkoxy, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, -$(CH_2)_m$-O-($C_1$-$C_{10}$ alkyl), aryl, and substituted aryl.

"$C_x$-$C_y$ alkyl" means a straight or branched chain hydrocarbon of between x and y carbon atoms.

"$C_x$-$C_y$ cycloalkyl" means a ring of between x and y carbon atoms having at least one fully saturated bond.

"Aryl" means one or more aromatic rings, each of 5 or 6 carbon atoms. Multiple aryl rings may be fused, as in naphthyl, or unfused, as in biphenyl.

"Substituted Aryl" having one or more non-interfering groups as substituents.

"Halo" means chloro, fluoro, iodo or bromo.

"Heterocycle" means one or more rings of 5, 6, or 7 atoms with or without unsaturation or aromatic character and at least one ring atom which is not carbon. Preferred heteroatoms include sulfur, oxygen, and nitrogen. Multiple rings may be fused, as in quinoline or benzofuran.

"Substituted heterocycle" means heterocycle with one or more side chains formed from non-interfering substituents.

II. General description of the tertiary amine combinatorial library:

The tertiary amine library of the invention is a diverse combinatorial library comprising individual tertiary amine library compounds represented by the general formulae (I) and (X):

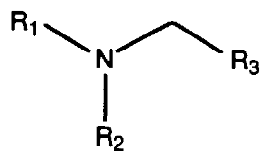

(I)

or

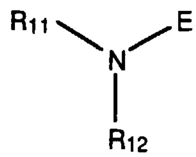

(X)

where $R_1$, $R_2$, $R_3$, $R_{11}$, $R_{12}$ and E are substituents defined below.

III. General description of the processes for making the tertiary amine combinatorial library of the invention:

Tertiary amines may be prepared by reductive amination with aldehydes or ketones.

Tertiary amines may also be prepared by nucleophilic substitution reactions of a secondary amine with an electrophile. For example, tertiary amines are made by the reaction of secondary amines with organohalides;

$$(CH_3CH_2)_2NH + CH_3CH_2Br \rightarrow (CH_3CH_2)_3N + HBr$$

or the reaction of secondary amines with esters of sulfonic acids;

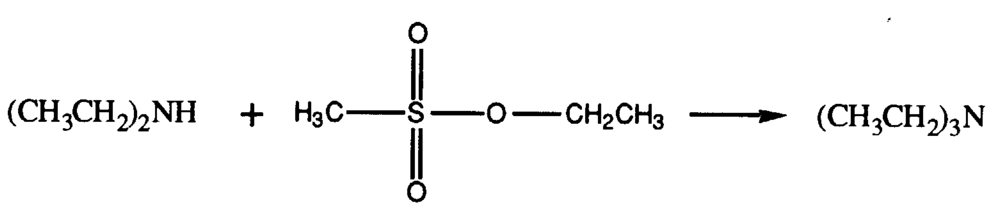

or hydroxylated tertiary amines may be made by the reaction of secondary amines and epoxides;

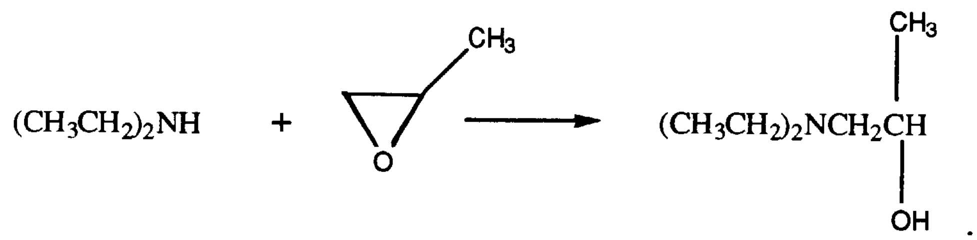

The various synthetic schemes for preparing individual tertiary amines as set out above are modified according to this invention to prepare tertiary amine combinatorial libraries.

This invention is particularly well suited as a general method for preparing a structurally diverse tertiary amine library. The final form of the library compounds in the tertiary amine library may be as a solute dissolved in a solvent (viz., the reaction medium) or the solvent may be removed and the final product retained as a powder, paste or oil.

The tertiary amine library compounds of this invention are non-peptide, substantially non-naturally occurring molecules having a molecular weight range of from about 100 to about 700.

The reaction zone for forming each tertiary amine library compound of this invention contains a solvent. The solvent reaction medium is typically a solvent not only for the library compound desired, but also for impurities such as; (i) unreacted reagent, and/or (ii) by-product(s). The impurities in the reaction mixture are generally soluble in the solution phase since they frequently have a molecular weight lower than or equal to the product and share broad solubility characteristics of the product.

One method of driving a multiplicity of tertiary amine forming reactions (viz., reductive amination, nucleophilic substitution) with diverse reactants to complete consumption of aldehyde or electrophile is to use a stoichiometric excess of amine reagent, preferably a large stoichiometric excess. For such methods the efficient removal of excess amine reagent may be accomplished with a solid supported amine reactive scavenger as taught herein.

Combinatorial techniques must be very robust to work well for highly diverse groups of reactants. The solid supported scavengers employed by the processes of this invention remove excess unreacted reactant in a manner readily adapted to the diversity of reagents employed in combinatorial chemistry, particularly parallel array synthesis.

A general scheme for the use of solid phase scavengers is as follows:

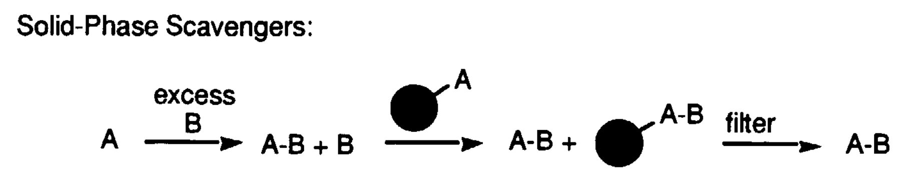


The solid supported scavenger is filtered from the liquid reaction media and the purified product A-B retained.

The utility of the method of the invention and the tertiary amine library created thereby is for developing new drugs. Pharmaceutical drug discovery relies heavily on studies of structure-activity relationships wherein the structures of discovered "lead compounds" are the basis for new drug development. The method of the invention systematically and simultaneously generates large numbers of diverse tertiary amine molecules useful as a source of lead compounds. The combinatorial tertiary amine libraries of the invention may be screened for pharmacologically active compounds using conventional screen protocols known in the art for any targeted disease state.

The following sections IV thru XI describe a combinatorial solution phase reductive amination process for making tertiary amine libraries

The following sections XII thru XVIII describe a combinatorial solution phase nucleophilic substitution process for making tertiary amine libraries.

REDUCTIVE AMINATION COMBINATORIAL PROCESS

IV. Description of the scavenger assisted solution phase reductive amination process of making tertiary amine combinatorial libraries of the invention.

This invention is a scavenger assisted combinatorial reductive amination process for preparing a library of compounds having a tertiary amine scaffold with three variable substituents, said compounds represented by the formula;

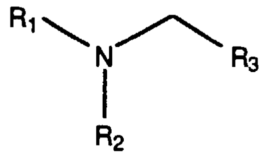

(I)

said process comprising the steps of:

a) adding to each reaction zone containing a liquid medium (n) equivalents of an aldehyde represented by the formula,

$$R_3CHO$$

where $R_3$ is a non-interfering substituent;
b) adding to each reaction zone of step (a) at least 1.1(n) equivalents of a solvent soluble secondary amine reactant represented by the formula:

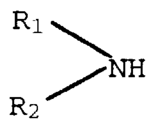

where $R_1$ and $R_2$ are independently selected from non-interfering substituents;
c) adding to each reaction zone a reducing agent and maintaining each reaction zone at a temperature and for a time sufficient to permit reductive amination of the secondary amine reactant;
d) adding to each reaction zone of step (c) a solid supported amine reactive scavenger represented by the formula;

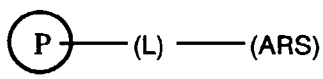

wherein;

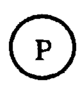

is a solid-support insoluble in the liquid reaction medium of the reaction zone, -(L)- is a divalent linking group, and (ARS) is an amine reactive substituent selected from isocyanate, isothiocyanate, or acyl halide; and adding said scavenger in an amount wherein the equivalents of (ARS) are at least equal to the excess equivalents of unreacted amine reactant used in step (b), and maintaining said reaction zone at a temperature and for a time sufficient to permit reaction of said excess amine reactant and said scavenger;
e) separating the solid supported scavenger from each reaction zone of step (d) and recovering each substantially purified tertiary amine library compound.

The "adding to each reaction zone" requirement for the secondary amine and aldehyde reactant in steps (a) and (b) means that different secondary amines and aldehydes may be added to each reaction zone in the library forming process, if desired. In one embodiment of the process of the invention, each combination of secondary amine and aldehyde added to each library reaction zone (e.g., wells of a wellplate) is different. Thus, the same amine may be added to each row of a wellplate apparatus (as per Fig. 1) and the same aldehyde reactant may be added to the same column of a wellplate apparatus to give a different combination of reactants in each well (viz., reaction zone) that will expectantly yield a different library compound. Alternatively, where it is desirable to have replicate samples, the same

combination of secondary amine and aldehyde may be added to different reaction zones.

V. Detail of Operation for the Reductive Amination Process - Step (a):

The reductive amination process is conducted using in step (a) an aldehyde;

$$R_3CHO ,$$

wherein $R_3$ is a non-interfering radical selected from $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, substituted phenyl, toluyl, xylenyl, biphenyl, $C_2$-$C_{12}$ alkoxyalkyl, $C_1$-$C_6$ alkylsulfinyl, $C_1$-$C_{10}$ alkylsulfonyl, $-(CH_2)_m$-O-($C_1$-$C_{10}$ alkyl), aryl, substituted aryl, substituted alkoxy, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, $-(CH_2)_m$-O-($C_1$-$C_{10}$ alkyl), aryl, and substituted aryl.

The aldehyde reactant $R_3CHO$ is preferably selected from aliphatic, aromatic, and heterocyclic aldehydes having a molecular weight of from 50 to 600.

Examples of aldehyde reactants suitable for use in the process of the invention are the following:

Aldehyde Reagents --

2-fluorenecarboxaldehyde
n-methylpyrrole-2-carboxaldehyde
furfural
5-nitro-2-furaldehyde
5-methylfurfural
5-acetoxymethyl-2-furaldehyde
5-hydroxymethyl-2-furaldehyde
benzaldehyde
2-bromobenzaldehyde
6-bromoveratraldehyde
2-fluorobenzaldehyde
pentafluorobenzaldehyde
2-chlorobenzaldehyde
2,4-dichlorobenzaldehyde
2-chloro-6-fluorobenzaldehyde
2,6-dichlorobenzaldehyde
o-anisaldehyde
2,3-dimethoxybenzaldehyde
2,3,4-trimethoxybenzaldehyde
2,4-dimethoxybenzaldehyde
2,4,5-trimethoxybenzaldehyde
2,4,6-trimethoxybenzaldehyde
2,5-dimethoxybenzaldehyde
2-ethoxybenzaldehyde
salicylaldehyde
3,5-dibromosalicylaldehyde
3-fluorosalicylaldehyde
3,5-dichlorosalicylaldehyde
3,5-diiodosalicylaldehyde
o-vanillin
3-ethoxysalicylaldehyde
2,3-dihydroxybenzaldehyde
2,3,4-trihydroxybenzaldehyde
4-(diethylamino)salicylaldehyde
2-hydroxy-4-methoxybenzaldehyde
4,6-dimethoxy-2-hydroxybenzaldehyde
2,4,6-trihydroxybenzaldehyde

5-bromosalicylaldehyde
5-chlorosalicylaldehyde
2-hydroxy-5-methoxybenzaldehyde
2,5-dihydroxybenzaldehyde
2-carboxybenzaldehyde
2-(trifluoromethyl)benzaldehyde
o-tolualdehyde
2,3-dimethyl-p-anisaldehyde
2,4-dimethylbenzaldehyde
mesitaldehyde
2,5-dimethylbenzaldehyde
2,5-dimethyl-p-anisaldehyde
3-cyanobenzaldehyde
3-bromobenzaldehyde
3-bromo-4,5-dimethoxybenzaldehyde
5-bromo-2-methoxybenzaldehyde
3-fluorobenzaldehyde
3-fluoro-p-anisaldehyde
3-chlorobenzaldehyde
3,4-dichlorobenzaldehyde
3,5-dichlorobenzaldehyde
3-phenoxybenzaldehyde
3-(3,4-dichlorophenoxy)benzaldehyde
3-(3,5-dichlorophenoxy)benzaldehyde
3-(3-(trifluoromethyl)phenoxy)benzaldehyde
3-(4-chlorophenoxy)benzaldehyde
3-(4-methoxyphenoxy)benzaldehyde
3-(4-tert-butylphenoxy)benzaldehyde
3-(4-methylphenoxy)benzaldehyde
m-anisaldehyde
4-acetoxy-3-methoxybenzaldehyde
3,4-dimethoxybenzaldehyde
3,4,5-trimethoxybenzaldehyde
4-benzyloxy-3-methoxybenzaldehyde
3,5-dimethoxybenzaldehyde
3-benzyloxybenzaldehyde
3-hydroxybenzaldehyde
3-hydroxy-4-methoxybenzaldehyde
3,4-dihydroxybenzaldehyde
3, 4, 5-trihydroxy benzaldehyde
3-(trifluoromethyl)benzaldehyde
m-tolualdehyde
3-methyl-p-anisaldehyde
4-cyanobenzaldehyde
4-bromobenzaldehyde
4-fluorobenzaldehyde
4-chlorobenzaldehyde
4-acetamidobenzaldehyde
4-dimethylaminobenzaldehyde
4-diethylaminobenzaldehyde
4-phenoxybenzaldehyde
4-acetoxybenzaldehyde
p-anisaldehyde
3-benzyloxy-4-methoxybenzaldehyde
4-benzyloxybenzaldehyde
4-ethoxybenzaldehyde
4-n-butoxybenzaldehyde
1-naphthaldehyde

2-methoxy-1-naphthaldehyde
2-hydroxy-1-naphthaldehyde
4-methoxy-1-naphthaldehyde
2-naphthaldehyde
1-pyrenecarboxaldehyde
3,4-dibenzyloxybenzaldehyde
n-ethyl-3-carbazolecarboxaldehyde
2-methyl-9-acridinecarboxaldehyde
pyrrole-2-carboxaldehyde
2-thiophenecarboxaldehyde
3-methylthiophene-2-carboxaldehyde
4-bromothiophene-2-aldehyde
5-bromo-2-thiophenecarboxaldehyde
5-nitrothiophene-2-carboxaldehyde
5-methyl-2-thiophenecarboxaldehyde
3-thiophenecarboxaldehyde
indole-3-carboxaldehyde
5-methoxyindole-3-carboxaldehyde
piperonal
6-nitropiperonal
2-pyridinecarboxaldehyde
6-methyl-2-pyridinecarboxaldehyde
3-pyridinecarboxaldehyde
4-pyridinecarboxaldehyde
3-quinolinecarboxaldehyde
4-quinolinecarboxaldehyde
4-hydroxybenzaldehyde
5-bromovanillin
5-iodovanillin
vanillin
syringaldehyde
3-ethoxy-4-hydroxybenzaldehyde
3,5-dimethyl-4-hydroxybenzaldehyde
4-biphenylcarboxaldehyde
4-(methylthio)benzaldehyde
methyl 4-formylbenzoate
4-carboxybenzaldehyde
4-trifluoromethylbenzaldehyde
4-isopropylbenzaldehyde
p-tolualdehyde
4-ethylbenzaldehyde
4-chloro-3-nitrobenzaldehyde
3,5-dinitro-2-hydroxybenzaldehyde
3-hydroxy-4-nitrobenzaldehyde
4-hydroxy-3-nitrobenzaldehyde
5-nitrovanillin
2-nitrobenzaldehyde
2,6-dinitrobenzaldehyde
6-nitroveratraldehyde
3-methoxy-2-nitrobenzaldehyde
2-chloro-6-nitrobenzaldehyde
3-nitrobenzaldehyde
5-chloro-2-nitrobenzaldehyde
2-chloro-5-nitrobenzaldehyde
5-hydroxy-2-nitrobenzaldehyde
5-nitrosalicylaldehyde
4-nitrobenzaldehyde
1,4-benzodioxan-6-carboxaldehyde

2,3-dichlorobenzaldehyde
3-ethoxy-4-methoxybenzaldehyde
3,5-bis(trifluoromethyl)benzaldehyde
2,3,6-trichlorobenzaldehyde
terephthalaldehyde monodiethylacetal
2,3-difluorobenzaldehyde
2,6-difluorobenzaldehyde
2,4-difluorobenzaldehyde
2,5-difluorobenzaldehyde
3,4-difluorobenzaldehyde
3,5-difluorobenzaldehyde
4-dimethylamino-1-naphthaldehyde
3-furaldehyde
3,4-dimethoxy-5-hydroxybenzaldehyde
2,3,5-trichlorobenzaldehyde
2,6-dimethoxybenzaldehyde
5-bromo-2,4-dimethoxybenzaldehyde
2,4-dimethoxy-3-methylbenzaldehyde
4-stilbenecarboxaldehyde
4-(3-dimethylaminopropoxy)benzaldehyde
2,4-dihydroxybenzaldehyde
3-chloro-4-fluorobenzaldehyde
2-methylindole-3-carboxaldehyde
4-hydroxy-3-methylbenzaldehyde
pyridoxal hydrochloride
2-(diphenylphosphino)benzaldehyde
2,4-dinitrobenzaldehyde
4-n-propoxybenzaldehyde
1-methylindole-3-carboxaldehyde
5-bromo-2-hydroxy-3-methoxybenzaldehyde
3-bromo-4-methoxybenzaldehyde
4-acetoxy-3,5-dimethoxybenzaldehyde
3,5-dihydroxybenzaldehyde
3-methoxy-4-(4-nitrobenzyloxy)benzaldehyde
2,3-(methylenedioxy)benzaldehyde
2-hydroxy-3-methoxy-5-nitrobenzaldehyde
2-cyanobenzaldehyde
5-ethyl-2-furaldehyde
4-tert-butylbenzaldehyde
3-tetrafluoroethoxybenzaldehyde
3-carboxybenzaldehyde
1-acetyl-3-indolecarboxaldehyde
4-(trifluoromethoxy)benzaldehyde
3-bromo-4-fluorobenzaldehyde
3-(trifluoromethoxy)benzaldehyde
2-chloro-4-fluorobenzaldehyde
5-(3-nitrophenyl)furfural
2-chloro-4-hydroxybenzaldehyde
2,3,4-trifluorobenzaldehyde
2-fluoro-3-(trifluoromethyl)benzaldehyde
2-fluoro-6-(trifluoromethyl)benzaldehyde
4-fluoro-2-(trifluoromethyl)benzaldehyde
2-quinolinecarboxaldehyde
4-(dibutylamino)benzaldehyde
5-(trifluoromethoxy)salicylaldehyde
3-fluoro-2-methylbenzaldehyde
3,5-dibenzyloxybenzaldehyde
5-(4-nitrophenyl)furfural

2-chloro-3-quinolinecarboxaldehyde
5-bromo-3-nitrosalicylaldehyde
2-chloro-5-(trifluoromethyl)benzaldehyde
5-bromo-2-furaldehyde
2,3,5,6-tetrafluorobenzaldehyde
4-methyl-5-imidazolecarboxaldehyde
2-benzyloxy-4,5-dimethoxybenzaldehyde
3,5-di-tert-butyl-2-hydroxybenzaldehyde
2,4-diethoxy-m-tolualdehyde
4-tert-pentylbenzaldehyde

VI. Detail of Operation for the Reductive Amination Process - Step (b):

The reductive amination process is preferably conducted using in step (b) a secondary amine;

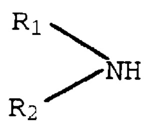

where $R_1$ and $R_2$ are independently selected from $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, substituted phenyl, toluyl, xylenyl, biphenyl, $C_2$-$C_{12}$ alkoxyalkyl, $C_1$-$C_6$ alkylsulfinyl, $C_1$-$C_{10}$ alkylsulfonyl, $-(CH_2)_m$-O-($C_1$-$C_{10}$ alkyl), aryl, substituted aryl, substituted alkoxy, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, $-(CH_2)_m$-O-($C_1$-$C_{10}$ alkyl), aryl, and substituted aryl.

The amine reactant;

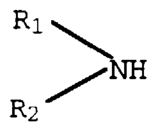

of step (b) is preferably selected from aliphatic, aromatic, and heterocyclic amines having a molecular weight of from 50 to 600.

Examples of suitable amine reactants for use in the process of the invention are the following:

Secondary Amine Reagents --

n-propylcyclopropanemethylamine
(n-butylamino)acetonitrile
n-methyl-beta-alaninenitrile
3-(benzylamino)propionitrile
3,3'-iminodipropionitrile
(r)-(-)-isoproterenol
(1r,2r)-(-) -pseudoephedrine
l-adrenaline
synephrine
2-(methylamino)ethanol
n-benzylethanolamine
2-(ethylamino)ethanol
diethanolamine
2-(propylamino)ethanol
heptamethyleneimine
n-isopropylcyclohexylamine
n-methylcyclohexylamine

n-ethylcyclohexylamine
allylcyclohexylamine
diisopropanolamine
n-methyl-d-glucamine
dibenzylamine
noreleagnine
propyleneimine
azetidine
n-omega-acetylhistamine
thiazolidine
3-pyrroline
2,5-dimethyl-3-pyrroline
pyrrolidine
l-prolinamide
l-prolinol
3-pyrrolidinol
n-omega-methyltryptamine
1-piperonylpiperazine
1,2,3,6-tetrahydropyridine
1-phenylpiperazine
1-(2-methoxyphenyl)piperazine
n-(3-trifluoromethylphenyl)piperazine
1-(4-fluorophenyl)piperazine
1-(4-nitrophenyl)piperazine
4-piperazinoacetophenone
1-ethoxycarbonylpiperazine
1-(4-chlorobenzhydryl)piperazine
n-methylpiperazine
1-benzylpiperazine
1-(pyrrolidinocarbonylmethyl)piperazine
n-isopropyl-1-piperazineacetamide
n-beta-hydroxyethylpiperazine
morpholine
2,6-dimethylmorpholine
thiomorpholine
1,4-dioxa-8-azaspiro[4.5]decane
piperidine
ethyl pipecolinate
2-methylpiperidine
2-piperidinemethanol
2-ethylpiperidine
2-piperidineethanol
n,n-diethylnipecotamide
ethyl nipecotate
nipecotamide
3-methylpiperidine
3,3-dimethylpiperidine
3,5-dimethylpiperidine
3-piperidinemethanol
4-hydroxypiperidine
4-hydroxy-4-phenylpiperidine
4-(4-chlorophenyl)-4-hydroxypiperidine
4-phenylpiperidine
ethyl isonipecotate
4-methylpiperidine
4-benzylpiperidine
1-(2-pyridyl)piperazine
2-(2-methylaminoethyl)pyridine

4-piperidinopiperidine
1-methyl-4-(methylamino)piperidine
decahydroquinoline
1,2,3,4-tetrahydroisoquinoline
hexamethyleneimine
dimethylamine
n-methylbenzylamine
n-methylphenethylamine
n'-benzyl-n,n-dimethylethylenediamine
methylaminoacetaldehyde dimethylacetal
n-methylpropargylamine
dipropargylamine
n-methylallylamine
diallylamine
diisopropylamine
n-isopropylbenzylamine
diisobutylamine
n-methyloctadecylamine
n-ethylmethylamine
n-ethylbenzylamine
diethylamine
n,n-dimethyl-n'-ethylethylenediamine
n,n-diethyl-n'-methylethylenediamine
n,n,n'-triethylethylenediamine
n-benzylglycine ethyl ester
di-sec-butylamine
methyl-n-propylamine
dipropylamine
n-methylbutylamine
n-butylbenzylamine
n-ethyl-n-butylamine
dibutylamine
di(2-ethylhexyl)amine
dipentylamine
di-n-hexylamine
di-n-octylamine
n-benzyl-2-phenylethylamine
9-(methylaminomethyl)anthracene
(s)-(+) -2-(methoxymethyl)pyrrolidine
2-methylaminomethyl-1,3-dioxolane
pindolol
n-ethylmethallylamine
dicyclohexylamine
1,4,5,6-tetrahydropyrimidine
n-(trimethylsilylmethyl)benzylamine
4,4-dimethyl-2-imidazoline
(s)-(+)-1-(2-pyrrolidinylmethyl)pyrrolidine
n,n,n'-trimethylethylenediamine
n,n,n'-trimethyl-1,3-propanediamine
tetramethylimino-bis-propylamine
(r)-(+)-n-benzyl-1-phenylethylamine
n-ethylisopropylamine
(s)-(+)-2-(anilinomethyl)pyrrolidine
(+/-)-nornicotine
2-(butylamino)ethanol
4-(ethylaminomethyl)pyridine
bis(2-methoxyethyl)amine
4-(1-pyrrolidinyl)piperidine

isonipecotamide
methylisopropylamine
n-methylhexylamine
(r)-(+)-n-methyl-1-phenylethylamine
3-(3-pyridylmethylamino)propionitrile
di-n-decylamine
piperidine thiocyanate
1-acetylpiperazine
n-methylhomopiperazine
1-ethylpiperazine
dl-adrenaline
trans-1-cinnamylpiperazine
(+)-pseudoephedrine
(-)-ephedrine
d-prolinol
2,6-dimethylpiperidine
(s)-(-)-n-benzyl-1-phenylethylamine
1,3,3-trimethyl-6-azabicyclo(3.2.1)octane
4-(4-bromophenyl) -4-piperidinol
(s)-(-)-n-methyl-1-phenylethylamine
n-methylhomoveratrylamine
(r)-(+)-atenolol
(s)-(-)-atenolol
1-hydroxyethylethoxypiperazine
demecolcine
n-allylcyclopentylamine
mitomycin c
di-beta-d-xylopyranosylamine
1-aza-12-crown-4
1-(formamidomethyl)-1h-benzotriazole
1-deoxy-1-(methylamino)-d-galactitol
1-deoxy-1-(octylamino)-d-glucitol
cytisine
(r)-(+)-3-pyrrolidinol
(+) -cis-2-benzylaminocyclohexanemethanol
(-) -cis-2-benzylaminocyclohexanemethanol
4-hydroxypyrimidine
(s)-3-pyrrolidinol

Other suitable secondary amines for use in the process of the invention are selected from the group represented by the formula:

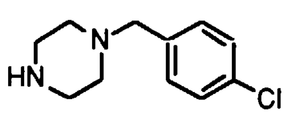

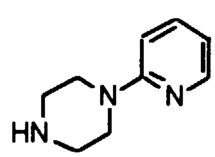

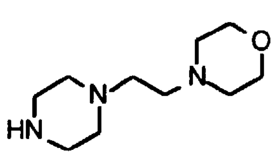

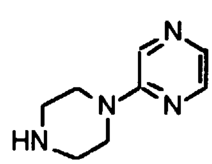

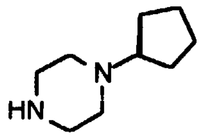

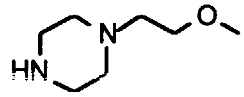

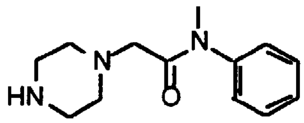

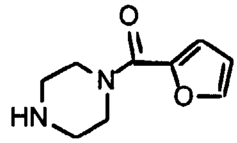

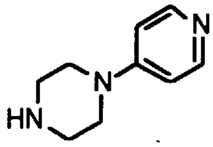

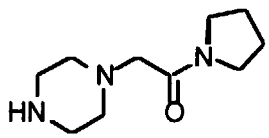

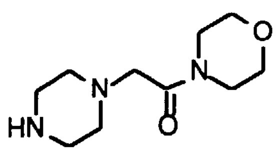

and

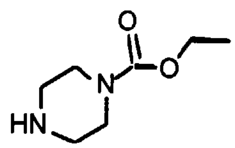

.

VII. Detail of Operation for the Reductive Amination Process - Step (c):

The reducing agent used in step (c) of the process may be any conventional reagents and attendant methods used for conducting reduction reactions; inclusive of H2/Pd, $H_2$ via high presure hydrogenation, $Na(OAc)_3BH$, lithium aluminum hydride, sodium borohydride, borohydride functional polymer, and cyanoborohydride functional polymer.

The cyanoborohydride functional polymer is a preferred reagent for use in the process of this invention. It is insoluble in the reaction media and may be conveniently removed in step (e) of this process by mechanical techniques (e. g., filtration). Cyanoborohydride functional polymer reducing agent may be prepared according to the procedure of Hutchins (see, Hutchins, R. O.; Natale, N. R.; Taffer, I. M. J. Chem. Soc. Chem. Comm. 1978, 1088).

VIII. Detail of Operation for the Reductive Amination Process - Step (d) :

The liquid reaction medium insoluble amine reactive scavenger added to each reaction zone of step (c) is represented by the formula;

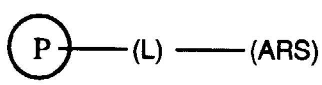


wherein;

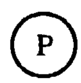


is a solid-support insoluble in the liquid medium of the reaction zone, -(L)- is a divalent linking group, and (ARS) is an

amine reactive substituent selected from isocyanate, isothiocyanate, or acyl halide.

Examples of organic solid supports are polymers such as polystyrene-divinylbenzene copolymer, polyacrylamide, cellulose and polystyrene. Examples of inorganic solid supports are silica gel, alumina, and controlled pore glass.

The group, -(L)-, is a linking group between the amine radical and the solid support and may be selected from a bond, or any divalent group. Preferably -(L)- is a divalent linking group of 1 to 40 atoms. Useful linking groups are selected from the following:

(bond),

$$-O-(CH_2)_x-$$

$$-S-(CH_2)_x-$$

$$-CH_2-(CH_2)_x-$$

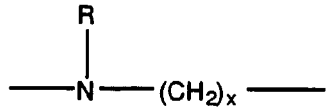

where R is hydrogen or $C_1$-$C_{10}$ alkyl, and x is an integer from 1 to 10.

The amine reactive substituent "(ARS)" substituent of the scavenger may be isocynate or isothiocyanate, represented by the formulae,

$$-NCO,$$

$$-NCS,$$

or an acyl halide selected from,

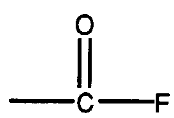

,

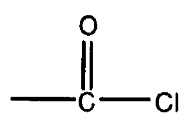

,

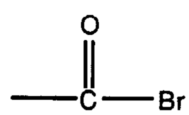

,

or

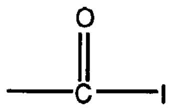 .

The amine reactive substituent on the scavenger readily reacts with the excess amine reagent used in step (b) of the process to covalently bind said excess reagent to the solid support and permit its simple removal as a solid phase. The effective available amine reactive content of the solid supported scavenger may be readily determined by conventional chemical analysis techniques.

The amount of solid supported amine scavenger,

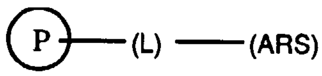

used in step (d) is based on the scavenger's available amine reactive functionality. The scavenger is added in at least an amount equal to the theoretical excess equivalents of unreacted amine reactant (viz., at least 0.1 equivalents used in step (b). For example, if 1.25 mmol. of piperidine constituting a 1.25 fold excess were used in step (b), then at least 0.25 mmol. of an (ARS) functional polymer would be added in step (d). Preferably, the solid supported scavenger is used in an amount that is from 1.25 to 5 times the theoretical excess equivalents of unreacted amine reagent.

The reaction zone is maintained at a temperature for a time sufficient to permit reaction of said excess secondary amine reactant and said amine reactive scavenger.

IX. Detail of Operation for the Reductive Amination Process - Step (e):

The final step in the tertiary amine library forming process of the invention is purification of the library compounds by separating the reacted and unreacted solid supported scavenger from the reaction medium of step (d) and recovering a solution of each substantially purified tertiary amine library compound.

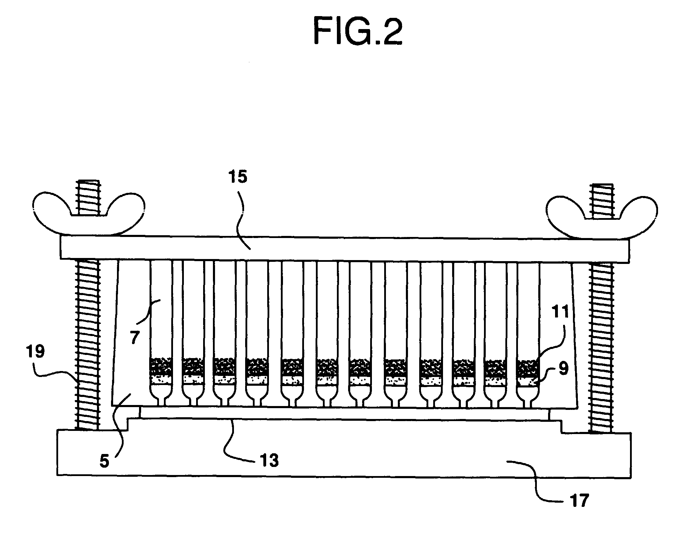

The separation of the solid supported scavenger from the library compound dissolved in the solvent phase of the reaction may be done by any conventional chemical or physical method. Preferred are physical methods which are applicable to all members of a diverse library. Such methods include; (i) filtration, (ii) centrifugation, (iii) decantation, and (iv) washing. Filtration is a particularly preferred form of purification. It is practiced by transporting each solution of library compound through a filter medium which retains the scavenger and transfers the solution phase into a separate vessel. An apparatus using filtration is depicted in Fig. 2, infra.

The purification last step of the process may optionally be supplemented by a solvent removal step in which the solute library compound is removed from its solvent by conventional processes known in the art; such as evaporation, distillation, freeze drying, salting out, solvent extraction, etc.

X. Other Details of the Reductive Amination Tertiary Amine Library making Process:

Reaction Medium - The reaction medium may be any liquid which has the following characteristics:

(1) the secondary amine and aldehyde reactants are capable of forming a reaction product which is substantially soluble in the reaction medium, and
(2) the solid supported scavenger, both in unreacted and reacted form, is substantially insoluble in the reaction medium.
(3) the solvent has sufficient polarity to be capable of stabilizing a charged species, such as iminium.
(4) the solvent is non-reactive with the reactants used in the library synthesis.

Typical reaction media useful in the processes of the invention are methanol, chloroform, methylene chloride, and acetonitrile.

The Reaction Zone - the process of the invention may be carried out in any vessel capable of holding the liquid reaction medium and having inlet and outlet means. Preferably the process of the invention is carried out in containers adaptable to parallel array syntheses. Most preferably, the tertiary amine library is formed in standard wellplates, such as the 96 well wellplate illustrated in Fig. 1 and/or the wellplate apparatus illustrated in Fig. 2. Each well may be filled by multiple delivery apparatus, automated or robotic apparatus, any of which may be either manually or computer controlled.

The diverse tertiary amine library of this invention may take the form of a plurality of wellplates, each wellplate

having wells containing a separate reaction product (library compound). In such cases, the library compounds are conveniently identified by their wellplate number and "x" column and "y" wellplate row coordinates.

A preferred technique for practicing the process of the invention is parallel array synthesis. With parallel array synthesis individual reaction products are prepared in each of multiple reaction zones. The amount of aldehyde and amine reactants introduced into each reaction zone will depend on the desired amount of each library compound that is needed for conducting biological assays, archival storage and other related needs. Typically, the desired amount of individual reaction product is from 1 microgram to 50 milligrams.

Proportions of reactants, reaction conditions:

The amount of aldehyde reactant in each reaction zone is represented by the symbol "(n)", where (n) represents the equivalents of aldehyde reactant.

The method of the invention contemplates solution phase reactions where a stoichiometric excess of secondary amine reactant is used. The amount of secondary amine used to insure an excess is defined as at least 1.1(n) and preferably a larger excess in the range of from 1.25(n) to 5(n), where the variable (n) is as previously defined. The stoichiometry of the reaction and calculation of equivalent weights of reagents may be done by reference to Scheme 1, infra. The 1.1 multiplier is used to insure at least a 10% stoichiometric excess of the secondary amine to drive the reaction to completion, thereby removing the aldehyde reactant from each reaction zone used to create the tertiary amine library. Thus, for example, if 1.25(n) - a 25% excess - of the secodary amine reactant is desired, then 10.6 mg of benzaldehyde would be used in step (a) of the process and 10.6 mg. of piperidine would be used in step (b) of the process.

The reaction zone is maintained at a temperature and for a time sufficient to permit reaction of the aldehyde and secondary amine reactants, that is, to complete consumption of the aldehyde and form an amount of tertiary amine library compound necessary to conduct biological assays to determine the efficacy of the prepared library compounds.

The time, temperature, and pressure of the combinatorial reaction zones used for the creation of library compounds are not critical aspects of the invention. Reaction times for a single step of the reaction are generally from 0.1 seconds to 24 hours, with times of 1 hour to 10 hours being most often used. The temperature of the reaction may be any temperature between the freezing point and the boiling point of the liquid reaction medium, but is generally between -10°C and +60°C, with 10°C to 40°C being preferred and ambient temperatures (about 20°C-30°C) being most preferred. The reactions may be conducted at subatmospheric pressure or superatmospheric pressure (viz., 60Kg./$m^2$ - 21000 Kg./$m^2$ absolute), but ambient atmospheric pressure (about 10330 Kg./$m^2$, absolute) is most often used.

Endpoint determination - The completion of the reaction between the aldehyde and secondary amine reactant may be determined by a number of conventional techniques. One method is to use thin layer chromatography to determine if the aldehyde reactant is substantially removed from the reaction zones.

Sequence of Operation - The addition of the aldehyde and secondary amine reactants to the reaction zone may take place in any order. For example, the secondary amine reactant may be initially added to the reaction zone followed by addition of the aldehyde reactant, or vice versa. Alternatively, the amine and aldehyde reactants may be simultaneously charged to each reaction zone.

The reaction zone is maintained at a temperature for a time sufficient to permit reaction of said excess secondary amine reactant and said scavenger. Typically, the reaction requires several minutes but the selection of reaction conditions that may be used is the same as set out in the preceding section IV.

XI. Reductive Amination Process - Reaction Scheme:

An illustrative reaction scheme for the reductive amination process for preparing libraries of tertiary amines is shown in Scheme 1, below:

Scheme 1

THE USE OF POLYSTYRENEBENZOYL CHLORIDE IN THE PURIFICATION OF TERTIARY AMINES

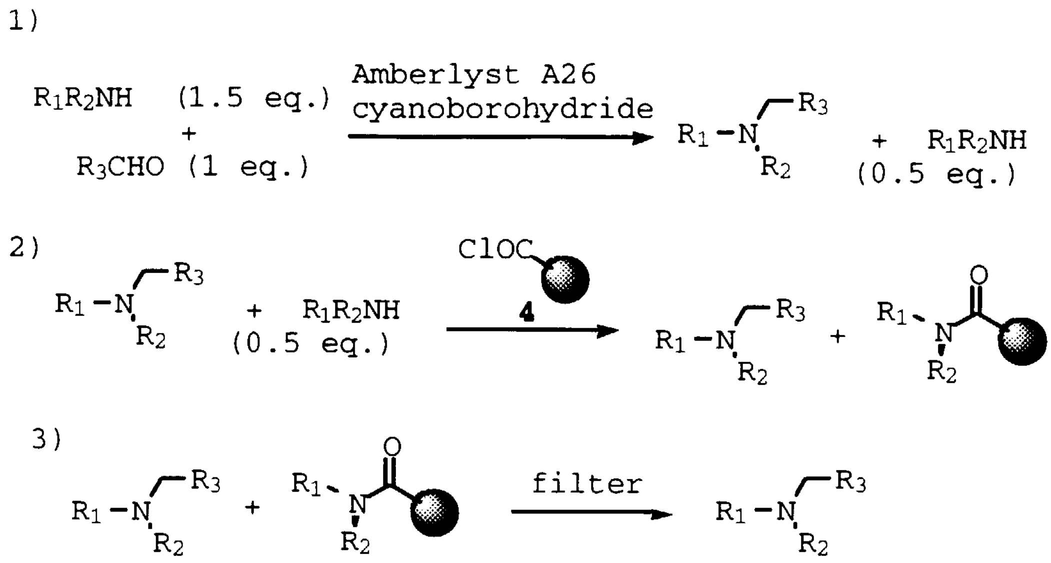


In the use of resin-bound benzoyl chloride **4** as a scavenger for secondary amines in the synthesis of tertiary amines (above), an aldehyde is combined with an excess of secondary amine in an acidic solvent system, such as 10% acetic acid in dichloroethane. To this solution is added an excess of Amberlyst A26 cyanoborohydride functional resin, and the mixture is allowed to react until no starting aldehyde remains (Step 1). When reaction is complete, an excess of polystyrene benzoyl chloride is added to the reaction mixture. This reacts with the remaining secondary amine to form an amide covalently bound to polystyrene (Step 2). When no secondary amine remains in solution, the mixture of product tertiary amine and resin is filtered through a cotton plug and the resin, retained by the plug, is rinsed free of any product by washing with additional solvent (Step 3).

NUCLEOPHILIC SUBSTITUTION COMBINATORIAL PROCESS

XII. Description of the scavenger assisted solution phase nucleophilic substitution process of making tertiary amine combinatorial libraries of the invention.

This invention is a scavenger assisted combinatorial nucleophilic substitution process for preparing a library of compounds having a tertiary amine scaffold with three variable substituents, said compounds represented by the formula (X);

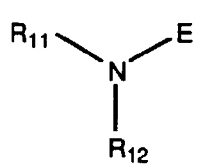

(X)

said process comprising the steps of:

a) adding to each reaction zone containing a liquid medium (n) equivalents of an electrophilic reactant, having electrophilic substituent, E;

If electrophilic reagent used in step (a) is an epoxide:

b) adding to each reaction zone of step (a) at least 1.1(n) equivalents of a solvent soluble secondary amine reactant represented by the formula:

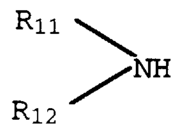

where $R_{11}$ and $R_{12}$ are independently selected from non-interfering substituents; or
If the electrophilic reagent used in step (a) is an organic halide or an ester of sulfonic acid:
b) adding to each reaction zone of step (a) containing a liquid medium;

(i) at least 1.1(n) equivalents of a solvent soluble secondary amine reactant represented by the formula:

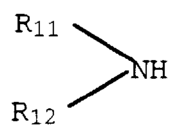

where $R_{11}$ and $R_{12}$ are independently selected from non-interfering substituents, and;
ii) a base in an amount sufficient to neutralize the acid, HX, formed;

c) adding to each reaction zone of step (b) a solid supported amine reactive scavenger represented by the formula;

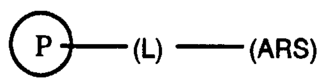

wherein;

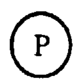

is a solid-support insoluble in the liquid medium of the reaction zone, -(L)- is a divalent linking group, and (ARS) is an amine reactive substituent selected from isocyanate, isothiocyanate, or acylhalide; and adding said scavenger in an amount wherein the equivalents of (ARS) are at least equal to the excess equivalents of unreacted amine reactant used in step (b), and maintaining said reaction zone at a temperature and for a time sufficient to permit reaction of said excess amine reactant and said scavenger;
d) separating the solid supported scavenger from each reaction zone of step (c) and recovering each substantially purified tertiary amine library compound.

The "adding to each reaction zone" requirement for the secondary amine and electrophilic reactant in steps (a) and (b) means that different secondary amines and electrophilic reactants may be added to each reaction zone in the library forming process, if desired. In one embodiment of the process of the invention, each combination of secondary amine and electrophilic reactant added to each library reaction zone (e.g., wells of a wellplate) is different. Thus, the same amine may be added to each row of a wellplate apparatus (as per Fig. 1) and the same electrophilic reactant may be added to the same column of a wellplate apparatus to give a different combination of reactants in each well (viz., reaction zone) that will expectantly yield a different library compound. Alternatively, where it is desirable to have replicate samples, the same combination of secondary amine and electrophilic reactant may be added to different reaction zones.

XIII. Detail of Operation for the Nucleophilic Substitution Process - Step (a):

The electrophilic reactant has an electrophilic substituent, E. The electrophilic reagent is preferably selected from an aliphatic or heterocyclic electrophile having a molecular weight of from 50 to 600, said electrophile selected from

the group consisting of an organic halide, an epoxide, and an ester of sulfonic acid.

Oraanic Halide Electrophilic reactant:

When an organic halide is selected as the electrophilic reactant it is preferably an organic halide represented by the formula;

$$R_{21}\text{-X}$$

where X is chloro, bromo, or iodo, and $R_{21}$ is a non-interfering substituent. The organic halide reactant is preferably selected from aliphatic, and heterocyclic organic halides amines having a molecular weight of from 50 to 600.

Most preferred are organic halides of the formula;

$$R_{21}\text{-x}$$

where $R_{21}$ is independently selected from $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, $C_2$-C12 alkoxyalkyl, $C_1$-$C_6$ alkylsulfinyl, $C_1$-$C_{10}$ alkylsulfonyl, $-(CH_2)_m$-O-($C_1$-$C_{10}$ alkyl), substituted alkoxy, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, C7-C12 aralkyl, $C_3$-$C_{10}$ cycloalkyl, $C_3$-$C_{10}$ cycloalkenyl, phenyl, and $-(CH_2)_m$-O-($C_1$-$C_{10}$ alkyl).

Examples of suitable organic halides useful in the tertiary amine combinatorial process of this invention are the following:

Organic Halides --

benzyl bromide
alpha-bromo-o-xylene
alpha-bromo-m-xylene
4-(tert-butyl)benzyl bromide
alpha-bromo-p-xylene
tert-butyl bromoacetate
methyl bromoacetate
benzyl bromoacetate
ethyl bromoacetate
2-bromoacetophenone
2-bromo-2'-methoxyacetophenone
2-bromo-2',4'-dimethoxyacetophenone
2-bromo-2',5'-dimethoxyacetophenone
3-methoxyphenacyl bromide
2-bromo-4'-methoxyacetophenone
2-bromo-4'-phenylacetophenone
2-bromo-4'-methylacetophenone
ethyl bromopyruvate
1-bromopinacolone
1-bromo-2-butanone
1-bromo-2,2-dimethoxypropane
1-bromo-2,2-dimethylpropane
bromoacetaldehyde dimethyl acetal
bromoacetaldehyde diethyl acetal
l-bromo-2-methylpropane
1-bromo-2-ethylbutane
2-ethylhexyl bromide
1-bromodecane
1-bromoundecane
2-bromoacetamide
iodoacetamide

4-(bromomethyl)phenylacetic acid phenacyl ester
isopropyl bromoacetate
5-bromo-2-methyl-2-pentene
3,4-difluorobenzyl bromide
2,5-difluorobenzyl bromide
3,5-bis(trifluoromethyl)benzyl bromide
2-bromo-2'-nitroacetophenone
3,5-difluorobenzyl bromide
2,4-bis(trifluoromethyl)benzyl bromide
8-bromo-1-octanol
4-(bromomethyl)phenylacetic acid
methyl (r)-(+) -3-bromo-2-methylpropionate
4-iodobutyl acetate
7-acetoxy-4-bromomethylcoumarin
4-bromomethyl-6,7-dimethoxycoumarin
2,4-difluorobenzyl bromide
methyl 2-(bromomethyl)acrylate
3-bromopropionaldehyde dimethyl acetal
(r)-(-)-3-bromo-2-methyl-1-propanol

Epoxide Electrophilic Reactant:

When an epoxide is selected as the electrophilic reactant it is preferably an epoxide having the general formula;

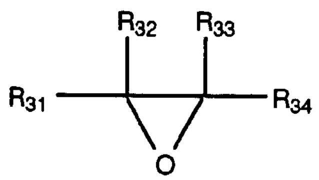

where $R_{31}$, $R_{32}$, $R_{33}$, and $R_{34}$ are independently selected from hydrogen or non-interfering substituents, with the proviso that at least one of $R_{33}$ and $R_{34}$ are hydrogen. Preferred epoxides are those where both $R_{33}$ and $R_{34}$ are hydrogen. The epoxide reactant is preferably selected from ethylene oxide or epoxides having a molecular weight of from 50 to 600 and may have aliphatic, aromatic, and heterocyclic substituents.

Most preferred are epoxides wherein $R_{31}$, $R_{32}$, $R_{33}$, and $R_{34}$ are independently selected from hydrogen and non-interfering substituents selected from the group consisting of $C_1$-$C_{10}$ alkyl, $C_2$-$C_{10}$ alkenyl, $C_2$-$C_{10}$ alkynyl, $C_1$-$C_{10}$ alkoxy, C7-C12 aralkyl, $C_7$-$C_{12}$ alkaryl, $C_3$-$C_{10}$ cycloalkyl, C3-C10 cycloalkenyl, phenyl, substituted phenyl, toluyl, xylenyl, biphenyl, C2-C12 alkoxyalkyl, $C_1$-$C_6$ alkylsulfinyl, $C_1$-$C_{10}$ alkylsulfonyl, $-(CH_2)_m$-O-($C_1$-$C_{10}$ alkyl), aryl, substituted aryl, substituted alkoxy, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, C7-C12 aralkyl, $C_7$-$C_{12}$ alkaryl, $C_3$-$C_{10}$ cycloalkyl, $C_3$-$C_{10}$ cycloalkenyl, phenyl, $-(CH_2)_m$-O-($C_1$-$C_{10}$ alkyl), aryl, and substituted aryl.

Examples of specific epoxides useful in the tertiary amine combinatorial library forming process of this invention are the following:

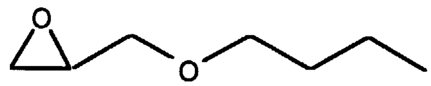

Butyl glycidyl ether

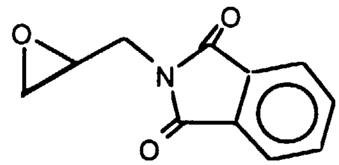

N-(2,3-Epoxypropyl)-phthalimide

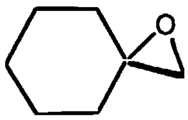

1-Oxaspiro (2,5) octane

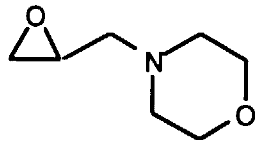

4-(2,3-Epoxypropyl) morpholine

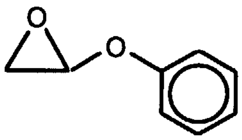

(±) -1,2-Epoxy-3-phenoxypropane

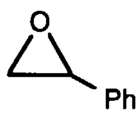

(2R,3R)-(+)-3-Phenyl glycidol

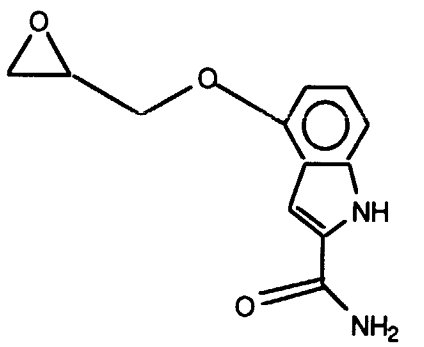

4-Glycidyloxy-2-Indole carboxamide

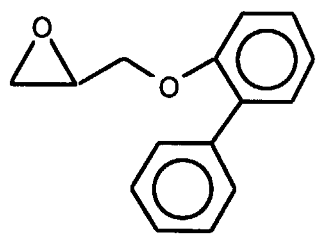

2-Biphenyl glycidyl ether

Ester of Sulfonic Acid Reactant:

When an ester of sulfonic acid is selected as the electrophilic reactant it preferably has the general formula;

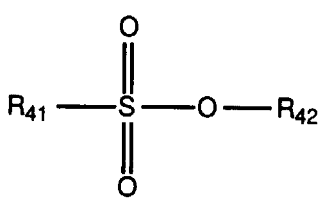

where R41 and R42 are independently aliphatic, aromatic, and heterocyclic non-interfering substituents and said ester has a molecular weight of from 110 to 600.

Most preferred are esters of sulfonic acid wherein $R_{41}$ is independently selected from $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_3$-$C_{10}$ cycloalkyl, $C_3$-$C_{10}$ cycloalkenyl, $C_2$-$C_{12}$ alkoxyalkyl, $C_1$-$C_6$ alkylsulfinyl, $C_1$-$C_{10}$ alkylsulfonyl, $-(CH_2)_m$-O-($C_1$-$C_{10}$ alkyl), substituted alkoxy, amidino, fluoroalkyl, aryloxyalkyl, heterocyclic radical, substituted heterocyclic radical, and nitroalkyl; where m is from 1 to 8. Preferred non-interfering radicals

are $C_1$-$C_{10}$ alkyl, $C_2$-$C_{10}$ alkenyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, $-(CH_2)_m$-O-($C_1$-$C_{10}$ alkyl), aryl, and substituted aryl.

Most preferred are esters of sulfonic acid wherein $R_{42}$ is independently selected from $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_3$-$C_{10}$ cycloalkyl, $C_3$-$C_{10}$ cycloalkenyl, $C_2$-$C_{12}$ alkoxyalkyl, $C_1$-$C_6$ alkylsulfinyl, $C_1$-$C_{10}$ alkylsulfonyl, $-(CH_2)_m$-O-($C_1$-$C_{10}$ alkyl), substituted alkoxy, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, and $-(CH_2)_m$-O-($C_1$-$C_{10}$ alkyl), .

Examples of sulfonic acid esters useful in the tertiary amine combinatorial process of this invention are the following:

Sulfonic Acid Esters --

ethyl trifluoromethanesulfonate
2,2,2-trifluoroethyl p-toluenesulfonate
2-chloroethyl-p-toluenesulfonate
1,3-propane sultone
5'-tosyladenosine
1,4-butane sultone
cyanomethyl benzenesulfonate
hexadecyl methanesulfonate
ethyl methanesulfonate
2-chloroethyl methanesulfonate
ethyl p-toluenesulfonate
trans-2-hydroxycyclohexyl p-toluenesulfonate
(2r)-(-)-glycidyl tosylate
(s)-(+)-2-methylbutyl methanesulfonate
(s)-(+)-2-methylbutyl p-toluenesulfonate
(s)-(+)-1-phenyl-1,2-ethanediol 2-tosylate
(2r)-(-)-glycidyl 3-nitrobenzenesulfonate
propargyl benzenesulfonate
2,2-dimethyl-1,3-dioxolan-4-ylmethyl p-toluenesulfonate
(r)-(-)-2,2-dimethyl-1,3-dioxolan-4-ylmethyl p-toluenesulfonate
(s)-(+)-2,2-dimethyl-1,3-dioxolan-4-ylmethyl p-toluenesulfonate
1,2:5,6-di-o-isopropylidene-3-o-(methylsulfonyl)-alpha-d-glucofuranose
ethyl l-2-((methylsulfonyl)oxy)propionate
(2s)-(+)-glycidyl tosylate
(2s)-(+)-glycidyl 3-nitrobenzenesulfonate
3-o-acetyl-6-o-benzoyl-5-o-(methylsulfonyl)-1,2-o-isopropylidene-alpha-d-glucofu
(r)-(-)-1-benzyloxy-3-(p-tosyloxy)-2-propanol
(s)-(+)-1-benzyloxy-3-(p-tosyloxy)-2-propanol
ethyl l-2-((trifluoromethylsulfonyl)oxy)propionate
2-(2-chloroethoxy)ethyl methanesulfonate
1-cyanoethyl p-toluenesulfonate

<u>XIV. Detail of Operation for the Nucleophilic Substitution Process - Step (b):</u>

In step (b) at least 1.1(n) equivalents of a solvent soluble secondary amine reactant represented by the formula:

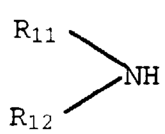

where $R_{11}$ and $R_{12}$ are independently selected from non-interfering substituents is added to each reaction zone of step (a).

The amine reactant used in step (b) of the nucleophilic process for preparing tertiary amines is the same as those

set out in Section VI, supra (for the reductive amination process), the disclosure of which is incorporated herein by reference. Thus, $R_{11}$ is the same as $R_1$ and $R_{12}$ is the same as $R_2$ described in Section VI.

When the electrophilic reagent used in step (a) is an organic halide or ester of a sulfonic acid a neutralizing agent (base) is required in the reaction zone after addition of secondary amine in step (b). The base added in step (b) is used to neutralize acid (e.g., HX) resulting from the reaction of step (b) as illustrated in Scheme 2. Conventional neutralizing agents such as alkali and alkaline-earth hydroxides may be used. However, it is preferred to use a resin bound base such as piperidinomethyl polystyrene as a solid phase scavenger of acid formed in the course of the reaction. Resin bound bases (or other solid neutralizing agents) may be conveniently removed from the reaction in the separation step (d), infra. The amount of acid anticipated to be formed may be is one mole of acid per mole of electrophilic reagent.

XV. Detail of Operation for the Nucleophilic Substitution Process - Step (c):

In step (c) solid supported amine reactive scavenger represented by the formula;

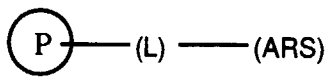

is added to each reaction zone of step (b). The scavenger is added to the reaction zone in a amount at least equal to the stoichiometric excess unreacted amine present in the reaction zone from the previous step. Preferably the solid supported amine reactive scavenger is added to the reaction zone in an amount in excess of the amine reactant to facilitate efficient amine removal.

XVI. Detail of Operation for the Nucleophilic Substitution Process - Step (d):

In step (d) the solid supported scavenger is separated from each reaction zone of step (c) and substantially purified tertiary amine library compound is recovered.

XVIII. Other Details of the Nucleophilic Substitution Tertiary Amine Library making Process:

Reaction Medium - The reaction medium may be any liquid which has the following characteristics:

(1) the secondary amine and aldehyde reactants are capable of forming a reaction product which is substantially soluble in the reaction medium, and
(2) the solid supported scavenger, both in unreacted and reacted form, is substantially insoluble in the reaction medium, and
(3) the reaction medium does not interfere with the reactants or the scavenger used in the process.

Typical reaction media useful in the processes of the invention are toluene, chloroform, methylene chloride, and acetonitrile, methanol, ethanol, isopropyl alcohol, or any of these used alone or in combination.

The Reaction Zone - the process of the invention may be carried out in any vessel capable of holding the liquid reaction medium and having inlet and outlet means. Preferably the process of the invention is carried out in containers adaptable to parallel array syntheses. Most preferably, the tertiary amine library is formed in standard wellplates, such as the 96 well wellplate illustrated in Fig. 1 and/or the wellplate apparatus illustrated in Fig. 2. Each well may be filled by multiple delivery apparatus, automated or robotic apparatus, any of which may be either manually or computer controlled.

The diverse tertiary amine library of this invention may take the form of a plurality of wellplates, each wellplate having wells containing a separate reaction product (library compound). In such cases, the library compounds are conveniently identified by their wellplate number and "x" column and "y" wellplate row coordinates.

A preferred technique for practicing the process of the invention is parallel array synthesis. With parallel array synthesis individual reaction products are prepared in each of multiple reaction zones. The amount of electrophilic reagent and amine reactants introduced into each reaction zone will depend on the desired amount of each library compound that is needed for conducting biological assays, archival storage and other related needs. Typically, the desired amount of individual reaction product is from 1 microgram to 50 milligrams.

The amount of electrophilic reactant in each reaction zone is represented by the symbol "(n)", where (n) represents the equivalents of electrophilic reactant.

The method of the invention contemplates solution phase reactions where a stoichiometric excess of secondary amine reactant is used. The amount of secondary amine used to insure an excess is defined as at least 1.1(n) and

preferably a larger excess in the range of from 1.25(n) to 5(n), where the variable (n) is as previously defined. The stoichiometry of the reaction and calculation of equivalent weights of reagents may be done by reference to Scheme 2, infra. The 1.1 multiplier is used to insure at least a 10% stoichiometric excess of the secondary amine to drive the reaction to completion, thereby removing the electrophilic reactant from each reaction zone used to create the tertiary amine library. Thus, for example, if 1.25(n) - a 25% excess - of the secondary amine reactant is desired, then 17.1 mg. of benzyl bromide would be used in step (a) of the process and 10.6 mg. of piperidine would be used in step (b) of the process.

The reaction zone is maintained at a temperature and for a time sufficient to permit reaction of the electrophilic and secondary amine reactants, that is, to complete consumption of the electrophilic reagent and form an amount of tertiary amine library compound necessary to conduct biological assays to determine the efficacy of the prepared library compounds.

The time, temperature, and pressure of the combinatorial reaction zones is the same as set out in Section X, supra., (for the reductive amination process) and is incorporated herein by reference. However, a preferred temperature range is from 20°C to 100°C.

Endpoint determination - The completion of the reaction between the electrophile and secondary amine reactant may be determined by a number of conventional techniques. One method is to use thin layer chromatography to determine if the electrophilic reactant is substantially removed from the reaction zones.

Sequence of Operation - The addition of the electrophile and secondary amine reactants to the reaction zone may take place in any order. For example, the secondary amine reactant may be initially added to the reaction zone followed by addition of the electrophile reactant, or vice versa. Alternatively, the amine and electrophilic reactants may be simultaneously charged to each reaction zone.

The amount of solid-supported scavenger added to the reaction product of step (b) is based on the scavenger's available amine reactive functionality. The scavenger is added in at least an amount equal to the theoretical excess equivalents of unreacted amine reactant (viz., at least 0.1 equivalents used in step (b). For example, if 1.25 mmol (10.6 mg.) of piperidine constituting a 25% (0.25 mmol) excess were used in step (b), then at least 250 mg. of polymer supported isocyanate having an isocyanate content of 1 meq/g. resin would be added in step (c). Preferably the solid supported scavenger is used in an amount that is from 1.25 to about 5 times the theoretical excess equivalents of unreacted secodary amine reactant.

The final step in the tertiary amine library forming process of the invention is purification of the library compounds by separating the reacted and unreacted solid supported scavenger from the reaction medium of step (c) and recovering a solution of each substantially purified tertiary amine library compound.

The separation of the solid supported scavenger from the library compound dissolved in the solvent phase of the reaction may be done by any conventional chemical or physical method. Preferred are physical methods which are applicable to all members of a diverse library. Such methods include; (i) filtration, (ii) centrifugation, (iii) decantation, and (iv) washing. Filtration is a particularly preferred form of purification. It is practiced by transporting each solution of library compound through a filter medium which retains the scavenger and transfers the solution phase into a separate vessel. An apparatus using filtration is depicted in Fig. 2, infra.

The purification last step of the process may optionally be supplemented by a solvent removal step in which the solute library compound is removed from its solvent by conventional processes known in the art; such as solvent evaporation, distillation, salting out, solvent extraction, and etc.

XVIII. Nucleophilic Substitution Process - Reaction Scheme:

The nucleophilic substitution process for preparing libraries of tertiary amines is shown in Scheme 2, below:

Scheme 2

USE OF POLYSTYRENE BENZYLISOCYANATE IN THE PURIFICATION OF TERTIARY AMINES

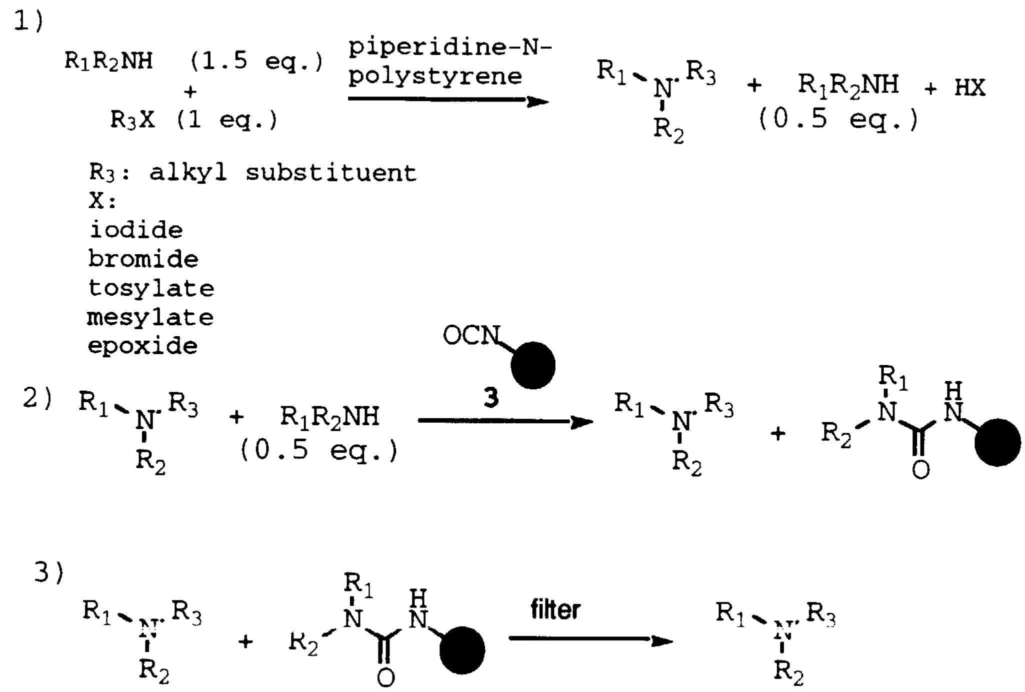


In the use of resin-bound benzylisocyanate **3** as a scavenger for secondary amines in the synthesis of tertiary amines (above), an electrophile (either an alkyl halide, sulfonate, or an epoxide) is combined with an excess of secondary aliphatic amine in a non-nucleophilic solvent such as methylene chloride or acetonitrile in the case of alkyl halides, sulfonates, or methanol in the case of epoxides (Step 1). For reactions with an alkyl halide, mesylate, or tosylate, a resin-bound base is added to neutralize acid produced in the course of the reaction, and the mixture is allowed to react until no starting electrophile remains. When reaction is complete, an excess of polystyrene-benzylisocyanate is added to the reaction (Step 2). This reacts with the remaining starting secondary amine to form a urea covalently bound to polystyrene. When no secondary amine remains in solution, the mixture of product tertiary amine and resin is filtered through a cotton plug and the resin, retained by the plug, is rinsed free of any product by washing with additional solvent (Step 3).

Other Utilities - Tertiary Amines:

Derivatives of tertiary amines (capable of being prepared by the method of this invention) have utility in a number of technological areas, as follows:

A) tertiary amines are catalysts for foundry mold resin curing, urethane catalysts, corrosion inhibitors in paint remover. B) Heat curable resin mixtures use tertiary amines with organic polyisocyanates - US Pat. No. 5,468,832. C) Alkyl substituted tertiary amines are useful for the preparation of low viscosity polymer-polyols - US Pat. No. 5,416,123.

XIX. Wellplate Apparatus containing library compounds prepared by the process of the invention:

The processes (both reductive amination and nucleophilic substitution) of making the tertiary amine libraries of the invention may be conveniently carried out in a wellplate apparatus such as illustrated in Fig. 1 and Fig. 2, hereinafter described. It is particularly advantageous to carry out the method of the invention in a standard wellplate apparatus such as a plastic 96 well microtiter plate.

Typically, the wellplate apparatus is in the form of a rigid or semi-rigid plate, said plate having a common surface containing openings of a plurality of vessels arranged in rows and columns. A standard form of wellplate apparatus is a rectangular plastic plate having 8 rows and 12 columns (total 96) of liquid retaining depressions on its surface. A wellplate apparatus may optionally have other elements of structure such as a top or cover (e.g., plastic or foil), a bottom in a form such as a plate or reservoir, clamping means to secure the wellplate and prevent loss of its contained compounds.

The sequence of operations to be used for library generation with the wellplate is as follows:

The wellplate apparatus of the invention:

A wellplate inoculated with the novel tertiary amine compounds of the invention is itself a new construct or apparatus which has particular utility in an assay kit used to discover lead compounds.
A suitable system of operation and related apparatus are made as follows:

1. Reaction zones are made by drilling 96 holes in the bottom of 96 deepwell titer plates and putting a porous frit in the bottom of each well.
2. The plate is put in a clamp assembly to seal the bottom of the wells.
3. Synthesis is begun by adding reagents to their assigned plate coordinates (reaction zone).
4. The plate is capped then tumbled to mix the reagents.
5. Solid supported scavenger is added to each reaction zone after completion of the reaction is shown by thin layer chromatography.
6. After sufficient reaction time the plate is removed from the clamp and the resin washed.
7. The solution containing product is filtered and the solution collected by transfer into another 96 well plate.
8. The reaction products (library compounds) are analyzed by thin layer chromatography.

FIG. 1 illustrates the top surface of a wellplate apparatus of the invention. The wellplate (3) is a plastic plate with 96 wells (depressions) capable of holding liquids. When used in the parallel array synthesis individual reaction products are prepared in each well and are labeled by the wellplate coordinates. The shaded circles in the Figure represent wells filled with tertiary amine library compounds prepared by the solution phase combinatorial processes of the invention. The compound at location (1), for example, is identified by the alphanumeric coordinate, "A6."

FIG. 2 illustrates a side view of a wellplate apparatus used in the Assay Kit of the invention. The wellplate (5) contains wells (7) with a filter (9) and liquid reaction medium containing scavenger (11). The wells have an outlet at bottom which is sealed by gasket (13) held in place by top cover (15) and bottom cover (17) maintained in position by clamp (19).

The libraries and compounds of the invention are prepared in the manner shown in the Schemes below.

XX. Assay Kits using wellplates with the library compounds of the invention:

This invention includes an assay kit for identification of pharmaceutical lead compounds. The assay kit comprises as essential parts, (i) wellplate apparatus (containing in its wells the tertiary amine library compounds of the invention), and (ii) biological assay materials.

The wellplate apparatus in the kit may comprise a set of wellplate apparatus such as illustrated in Fig. 1. The tertiary amine library compounds contained in each wellplate may be prepared by either the reductive amination process or the nucleophilic substitution process taught herein. Preferably the wellplate apparatus has the form of a standard 96 well microtiter plate.

The assay kit also contains biological assay materials. These biological assay materials are generally in vitro tests known to be predictive of success for an associated disease state. Illustrative of biological assay materials useful in the kit of this invention are those required to conduct the following assays:

In vitro assays:

Enzymatic Inhibition
Receptor-ligand binding
Protein-protein Interaction
Protein-DNA Interaction

Cell-based, Functional assays:

Transcriptional Regulation
Signal Transduction/ Second Messenger
Viral Infectivity

Add, Incubate, & Read assays:

Scintillation Proximity Assays

Angiotensin II SPA receptor binding assay
Endothelin converting enzyme [$^{125}$I] SPA assay
HIV proteinase [$^{125}$I] SPA enzyme assay
Cholesteryl ester transfer protein (CETP) [$^{3}$H] SPA assay

Fluorescence Polarization Assays
Fluorescence Correlation Spectroscopy
Colorimetric Biosensors
$Ca^{2+}$-EGTA Dyes for Cell-based assays
Reporter Gene Constructs for cell based assays

Luciferase, green fluorescent protein,
β-lactamase

Electrical cell impedance sensor assays

EXAMPLES - REDUCTIVE AMINATION

Reductive Amination Using Cyanoborohydride Resin:

To each of the twelve columns in a 96 well teflon plate is added 0.15 mmol of secondary amine, one amine per column, as a stock solution in 10% acetic acid in dichloroethane (0.5M, 0.3mL). To each of the eight rows is added neat 0.1 mmol of aldehyde, one aldehyde per row. To each of the 96 wells is added 40-50 mg cyanoborohydride resin (3.3 mmol $CNBH_3^-$/g resin, 0.15 mmol). The plate is sealed and shaken for approximately 24 hours at room temperature. To each well is added 25-35 mg of acid chloride resin (2.5 mmol $Cl^-$ /g resin) and 0.lmL dichloromethane. The plate is sealed and shaken for 5 hours. The product solutions are filtered through the frits in the bottom of the wells directly into a pre-tared 96 well microtitre plate. The solvent is removed and the plate re-weighed to determine an average mass per well. An average yield is calculated by dividing the average mass per well by the average molecular weight of the tertiary amine products.

The following products were representative of those prepared in this Example:

1-(2-methoxyethyl)-piperazine and 2-furaldehyde yielded 1-(2-methoxyethyl)-4-(2-furylmethyl)-piperazine
MS (m/e): 224 (M+)
1-benzylpiperazine and 4-fluorobenzaldehyde yielded 1-benzyl-4-(4-fluorophenylmethyl)-piperazine
MS (m/e): 284 (M+)
1-(4-fluorophenyl)-piperazine and 4-methylthiobenzaldehyde yielded 1-(4-fluorophenyl)-4-(4-methylthiophenylmethyl)-piperazine
MS (m/e): 316 (M+)
1-(4-methoxyphenyl)-piperazine and 3-phenoxybenzaldehyde yielded 1-(4-methoxyphenyl)-4-(3-phenoxyphenylmethyl)-piperazine
MS (m/e): 375 (M+)
1-(2-dimethylaminoethyl)-piperazine and 1-napthaldehyde yielded 1-(2-dimethylaminoethyl)-4-(1-napthylmethyl)-piperazine
MS (m/e): 297

EXAMPLES - NUCLEOPHILIC SUBSTITUTION

Procedures for the synthesis of Polymer Bound Isocyanate Resin

Isocyanate Resin (3): (Note: this material has been reported in the literature: Rebek, J.; Brown, D.; Zimmerman,

S. *J. Am. Chem. Soc.* 1975, *97*, 4407) To a stirring solution (a stirring paddle was used) of 50 g (61 mmol, 1 equiv.) of aminomethyl polystyrene (Advanced Chemtech, 100-200 mesh, 1%DVB, 1.16 mmol/g) in 800 mL of anhydrous toluene was added in one portion 193 mL (366 mmol, 6.0 equiv.) of a 1.9 M solution of phosgene in toluene (Fluka). The reaction was strirred 10 min. and then 67 mL (482 mmol, 7.9 equiv.) of triethylamine was added via syringe which immediately resulted in the formation of a white precepitate. The reaction was stirred overnight, filtered under a stream of nitrogen and washed with methylene chloride (1.0 L). The crude resin (contaminated with triethylamine hydrochloride) was added to 500 mL of CH2C12, stirred 15 min. and filtered under a stream of nitrogen. This step was then repeated once more. The resin was then washed with ether (500 mL) and dried overnight in a vacuum oven with light heating (40-50_C) to give 49 g of a light yellow resin. IR (KBr) 2252cm$^{-1}$. Elemental Analysis: C, 87.33; H, 7.56; N, 1.80 (theoretical 1.71); Cl, None.

Solution Phase Opening of Epoxides with Amines:

A secondary amine ( 0.10 mmol, 2 equiv.) and an epoxide (0.05 mmol, 1 equiv.) in 1 mL of methanol were shaken for 2 days at ambient temperatures in a 4.0 mL glass vial with a Teflon lined cap. The reaction mixture was then heated overnight at 65_C, cooled and diluted with 2 mL of CH2C12. Polystyrene benzylisocyanate (150-250 mg, 0.17 -0.29 mmol, >3 equiv.) was added and the reaction mixture was shaken overnight. Removal of the spent resin by filtration through a plug of cotton in a pipette (using 1 mL of $CH_2Cl_2$ as a wash) followed by evaporation of the solvent under nitrogen or under reduced pressure in a speed vac gave amino alcohols in average yields of 80% with average HPLC purities of 80%. Over 1000 compounds were combinatorically generated using this method.

Purer samples were obtained by treatment of the crude product with a SCX ion exchange column (500 mg, 3 mL column size, Varian). The crude product was redissolved in 1-2 mL of a 10% MeOH / $CH_2Cl_2$ solution and applied directly onto the ion exchange column. The solvent was slowly pulled through using light vacuum over approximately 1-1.5 min., washed 2-3 X with 2 mL of MeOH and then eluted from the column using three 2 mL portions (6 mL total) of a 20% solution of sat. $NH_3$·MeOH / $CH_2Cl_2$ Concentration under vacuum in a speed vac provided materials with average HPLC purities >90%.

Alkylation Of Secondary Amines With Iodides Or Bromides:

Iodide (0.02 mmol) was dissolved in 150 mL methylene chloride in a 4 mL screw cap vial, and a secondary amine (0.04 mmol) was added neat. Resin-bound tertiary amine (18-20 mg, 0.3 mmol) was then added and the reaction mixture was shaken at room temperature for 3 days, at which time no halide remained by TLC. An additional 1 mL of methylene chloride was added, followed by 50 mg (0.04 meq.) resin-bound isocyanate (loading 0.8meq./g) , and the resulting mixture was shaken for 6 hours, at which time no starting secondary amine remained by TLC. The reaction was filtered through a cotton plug, and the solid residue was rinsed with more methylene chloride. The solvent was evaporated to give the product tertiary amine.

Note: for alkyl bromides, typical solvent is acetonitrile rather than methylene chloride (although the reaction is still diluted with 1 mL methylene chloride), and the reaction typically requires heating at 50-60°C.

Examples of tertiary amines prepared by the process of the invention are as follows:

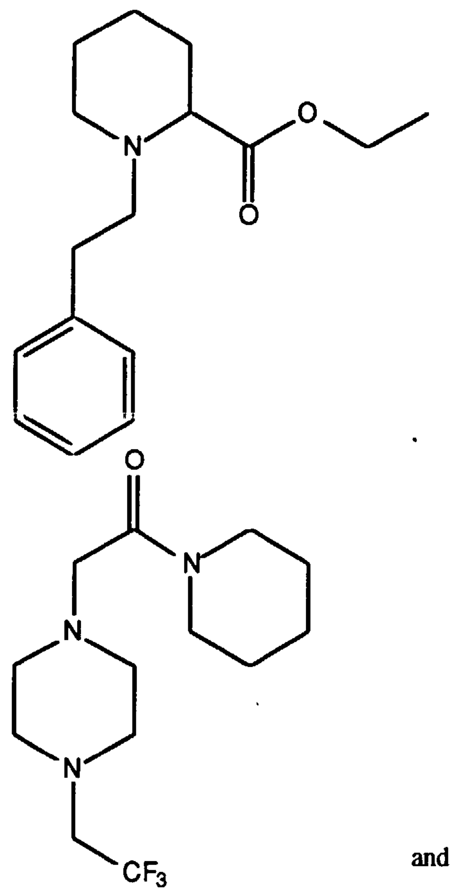

.

and

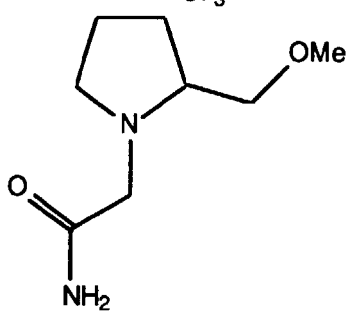

**Claims**

1. A scavenger assisted solution phase combinatorial reductive amination process for preparing a library of compounds having a tertiary amine scaffold with three variable substituents, wherein each library compound is made in a separate reaction zone and is represented by the formula (I);

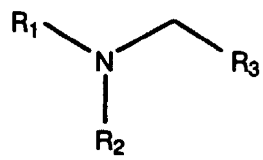 (I)

said process comprising the steps of:

a) adding to each reaction zone containing a liquid medium (n) equivalents of an aldehyde represented by the formula,

$$R_3CHO$$

where $R_3$ is a non-interfering substituent;

b) adding to each reaction zone of step (a) at least 1.1(n) equivalents of a solvent soluble secondary amine reactant represented by the formula:

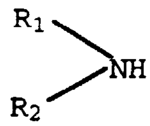

where $R_1$ and $R_2$ are independently selected from non-interfering substituents and maintaining each reaction zone at a temperature and for a time sufficient to permit reaction of the aldehyde of step (a) and said secondary amine;

c) adding to each reaction zone a reducing agent and maintaining each reaction zone at a temperature and for a time sufficient to permit reductive amination of the secondary amine reactant;

d) adding to each reaction zone of step (c) a solid supported amine reactive scavenger represented by the formula;

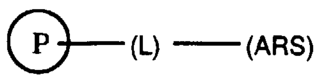

wherein;

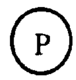

is a solid-support insoluble in the liquid reaction medium of the reaction zone, -(L)- is a divalent linking group, and (ARS) is an amine reactive substituent selected from isocyanate, isothiocyanate, or acyl halide; and adding said scavenger in an amount wherein the equivalents of (ARS) are at least equal to the excess equivalents of unreacted amine reactant used in step (b), and maintaining said reaction zone at a temperature and for a time sufficient to permit reaction of said excess amine reactant and said scavenger;

e) separating the solid supported scavenger from each reaction zone of step (d) and recovering each substantially purified tertiary amine library compound.

**2.** The process of claim 1 wherein from 1.25(n) to 5(n) equivalents of secondary amine reactant is used per equivalent of aldehyde reactant.

**3.** The library of tertiary amine compounds prepared by the process of claim 1.

**4.** The individual tertiary amine library compounds in the tertiary amine library of claim 3.

**5.** A scavenger assisted solution phase combinatorial nucleophilic substitution process for preparing a library of compounds having a tertiary amine scaffold with three variable substituents, wherein each library compound is made in a separate reaction zone and is represented by the formula (X);

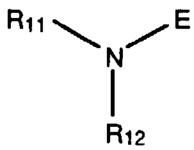 (X)

said process comprising the steps of:

a) adding to each reaction zone containing a liquid medium (n) equivalents of an epoxide electrophilic reactant, having electrophilic substituent, E;

b) adding to each reaction zone of step (a) at least 1.1(n) equivalents of a solvent soluble secondary amine reactant represented by the formula:

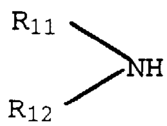

where $R_{11}$ and R12 are independently selected from non-interfering substituents;

c) adding to each reaction zone of step (b) a solid supported amine reactive scavenger represented by the formula;

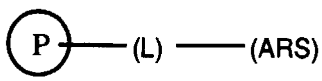

wherein;

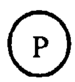

is a solid-support insoluble in the liquid medium of the reaction zone, -(L)- is a divalent linking group, and (ARS) is an amine reactive substituent selected from isocyanate, isothiocyanate, or acylhalide; and adding said scavenger in an amount wherein the equivalents of (ARS) are at least equal to the excess equivalents of unreacted amine reactant used in step (b), and maintaining said reaction zone at a temperature and for a time sufficient to permit reaction of said excess amine reactant and said scavenger;

d) separating the solid supported scavenger from each reaction zone of step (c) and recovering each substantially purified tertiary amine library compound.

**6.** A scavenger assisted solution phase combinatorial nucleophilic substitution process for preparing a library of compounds having a tertiary amine scaffold with three variable substituents, wherein each library compound is made in a separate reaction zone and is represented by the formula (X);

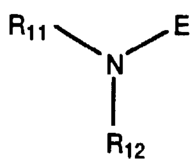 (X)

said process comprising the steps of:

a) adding to each reaction zone containing a liquid medium (n) equivalents of an electrophilic reactant, having electrophilic substituent, E;
b) adding to each reaction zone of step (a) containing a liquid medium;

(i) at least 1.1(n) equivalents of a solvent soluble secondary amine reactant represented by the formula:

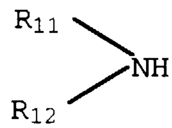

where $R_{11}$ and $R_{12}$ are independently selected from non-interfering substituents, and;
ii) a base in an amount sufficient to neutralize the acid formed;

c) adding to each reaction zone of step (b) a solid supported amine reactive scavenger represented by the formula;

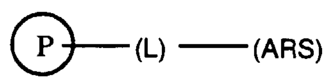

wherein;

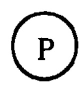

is a solid-support insoluble in the liquid medium of the reaction zone, -(L)- is a divalent linking group, and (ARS) is an amine reactive substituent selected from isocyanate, isothiocyanate, or acylhalide; and adding said scavenger in an amount wherein the equivalents of (ARS) are at least equal to the excess equivalents of unreacted amine reactant used in step (b), and maintaining said reaction zone at a temperature and for a time sufficient to permit reaction of said excess amine reactant and said scavenger;
d) separating the solid supported scavenger from each reaction zone of step (c) and recovering each substantially purified tertiary amine library compound.

**7.** The process of claim 5 or 6 wherein from 1.25(n) to 5(n) equivalents of secondary amine reactant is used per equivalent of electrophilic reactant.

**8.** The process of claim 5 and 6 wherein the scavenger, of step (c) has a divalent linking group -(L)- of 1 to 40 atoms, and the group (ARS) is an isocyanate.

**9.** An assay kit for identification of pharmaceutical lead compounds, comprising biological assay materials and wellplate apparatus;
wherein the improvement comprises using as wellplate apparatus a wellplate containing in each well library compound of a diverse combinatorial tertiary amine library prepared by the processes of claims 1 or 5 or 6.

**10.** Wellplate apparatus suitable as a replaceable element in an automated assay machine wherein the improvement comprises,
using as the wellplate apparatus a diverse tertiary amine combinatorial wellplate, wherein each well independently contains a tertiary amine library compound prepared by the processes of claim 1 or 5 or 6.

FIG.1

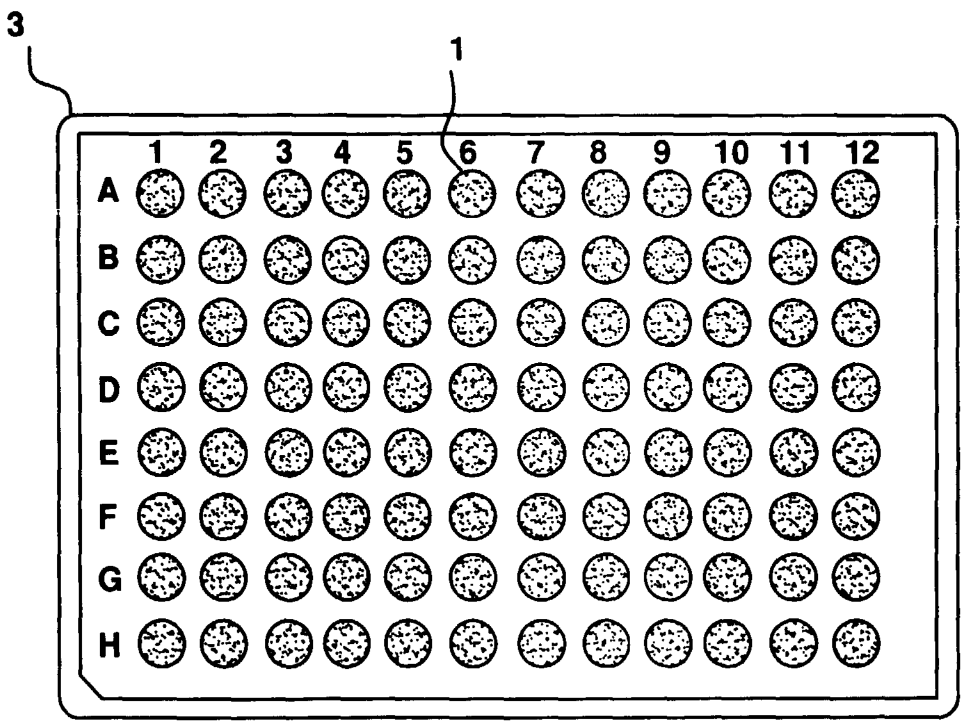

FIG.2